# EUROPEAN PATENT APPLICATION

(11) **EP 1 454 626 A1**
(43) Date of publication of application: **08.09.2004**
(21) Application number: 02790789.8
(22) Date of filing: 16.12.2002
(51) Int. Cl.: A61K 35/84, A61K 31/07, A61K 31/203, A61P 35/00, A61P 43/00

(54) **METHOD OF INDUCING APOPTOSIS AND COMPOSITIONS THEREFOR**

(30) Priority: 14.12.2001 JP 2001382300
(71) Applicant: Sundory Co., Ltd., Sakai-shi, Osaka 591-8023 (JP); Kyowa Engineering Co., Ltd., Chiyoda-ku, Tokyo 102-0074 (JP)
(72) Inventor: MORI, Takeshi, Nagoya-shi, Aichi 463-0048 (JP)
(74) Representative: Khoo, Chong-Yee
(86) International application number: PCT/JP2002/013146
(87) International publication number: WO 2003/051382

(57) **Abstract**

A novel composition and health food having a good pharmacological activity and which can be readily absorbed through an alimentary canal are provided. The composition induced apoptosis of cells and includes an agaricus extract. The composition may further comprise a pharmaceutically acceptable carrier. Typically, the cells are cancer cells. The composition may further include a differentiation induction agent. Preferably, the differentiation induction agent is a vitamin A derivative. The vitamin A derivative may be tretinoin. The agaricus extract may include a main fraction eluted chromatographically of a molecular weight of 100 to 2000, which is obtained by the steps of extraction with hot water from a fruiting body of agaricus, dialyzing the extract and performing a chromatography process on the dialyzate.

## Description

### TECHNICAL FIELD

The present invention relates to amethod for inducing apoptosis which comprises a substance obtained by an extraction process from agaricus and a composition for the same.

### BACKGROUND ART

Apoptosis (programmed cell death) has an important roll in morphogenesis in ontogeny, in maintaining homeostasis of adult tissues, and the like, such as, rejuvenescence of an epithelial cell of the alimentary canal, cytolysis of T cells which recognize an autoantigen, and excessive formation of cells followed by cell death at specific sites to form a central nervous system with a specific layered structure. In general, death of a cell by apoptosis requires new protein synthesis. A gene which induces cell death is called a suicide gene.

In general, cancer cells achieve autonomous replication through some kind of gene mutation and continue to replicate themselves infinitely. Recently, studies on apoptosis have been advanced, and its molecular mechanism is becoming clear. HL60 cell line, which is a promyelocytic leukemia cell line, is widely used as preferable model in screening for apoptosis inducing substances. The HL60 cell line is also a suitable model in screening for a differentiation inducing substance. When a HL60 cell line is induced to differentiate, its growth is suppressed and the tumor producing effect is lost by terminal differentiation (out of cancerous stage). As treatments for cancer, induction or apoptosis and differentiation induction are considered to be effective measures. Certain kinds of anticancer drugs and retinoic acid derivatives have already been applied clinically. A number of substances derived from a plant which have an antitumor effect have been found. In recent years, a taxane type anticancer drug, a vinca alkaloid type anticancer drug, Camptothecin (alkaloid) and the like have been developed and have achieved clinical results.

A basidiomycete of the family Agaricaceae, Agaricus blazei Murill, is generally called an agaricus mushroom and is known to have antitumor activity. Powder or various extracts of agaricus are taken orally as health foods. With respect to ingredients from agaricus , there have been a number of reports regarding activities of macromolecular substances such as polysaccharides, including β-D-glucan, and proteins, or the like from agaricus. However, in general, a macromolecular substance such as protein or polysaccharide is not always well absorbed through the alimentary canal. Hitherto, it was generally known that cell body ingredients of agaricus are difficult to digest. Thus, there is a demand for a substance derived from agaricus which has good bioactivity and is orally administrable as a medicament and health food.

### DISCLOSURE OF THE INVENTION

Cell growth suppression activity, differentiation induction activity, and apoptosis induction activity of a material derived from agaricus are confirmed, and a material derived from agaricus which has good bioactivity and is orally administrable as a medicament and health food is provided.

The present invention relates to a composition for inducing apoptosis of cells and the composition includes an agaricus extract.

Preferably, the composition further comprises a pharmaceutically acceptable carrier.

Preferably, the cells are cancer cells.

Preferably, the composition further comprises a differentiation induction agent.

Preferably, the differentiation induction agent is a vitamin A derivative.

Preferably, the vitamin A derivative is tretinoin.

The vitamin A derivative may also be carotenoid.

The present invention also relates to a method for inducing apoptosis of cells, and the method comprises a step of administering a composition including an agaricus extract to a subject.

Preferably, the composition further comprises a pharmaceutically acceptable carrier.

Preferably, the cells are cancer cells.

Preferably, the method further comprises a differentiation induction agent.

Preferably, the differentiation induction agent is a vitamin A derivative.

Preferably, the vitamin A derivative is tretinoin.

Typically, the agaricus extract may be obtained by extraction with hot water from a fruiting body of agaricus.

The agaricus extract may include a main fraction eluted chromatographically of a molecular weight of 100 to 2000 obtained by the steps of extraction with hot water from a fruiting body of agaricus, dialyzing the extract, and performing a chromatography process on the dialyzate.

Alternately, the agaricus extract may include, as an effective ingredient, a dialyzate obtained by the steps of extraction with hot water from a fruiting body of agaricus, mixing the obtained extract with ethanol and centrifuging to separate precipitate and supernatant, mixing the supernatant with ethanol and centrifuging to separate precipitate and supernatant, and dissolving the precipitate into diluted water to perform dialysis.

The agaricus extract may as appropriate be in the form of mixed with a pharmaceutically acceptable carrier. Pharmaceutically acceptable carriers are known to those skilled in the art and include, for example, the following carriers but not limited to these: buffers such as Ringer's solution, Hank's balanced salt solution, or buffered physiological saline; fatty acids such as sesame oil; synthetic fatty acid esters such as ethyl oleate or triglycerides; saccharides such as lactose, sucrose, mannitol, sorbitol; starches derived from vegetables such as corn, wheat, rice, or potato; cellulose such as methylcellulose, hydroxypropylmethylcellulose, or sodium carboxymethylcellulose; rubber such as gum arabic or tragacanth; proteins such as gelatin or collagen; cross-linked polyvinyl pyrrolidone, agar, alginic acid or salts thereof, or the like.

The present invention also relates to use of an agaricus extract for preparing a composition for inducing apoptosis of cancer cells.

### BRIEF DESCRIPTION OF THE DRAWINGS

Figure **1** is a diagram which shows an effect of the present invention, showing growth suppression on leukemia cell lines (HL-60 lines) in the presence of an agaricus extract (ABMK-22);
Figure **2** is a diagram which shows an effect of the present invention, showing growth suppression on leukemia cell lines (HL-60 lines) in the presence of an agaricus extract (ABMK-22);
Figure **3** is a diagram which shows an effect of the present invention, showing growth suppression on leukemia cell lines (HL-60 lines) in the presence of an agaricus extract (ABMK-22);
Figure **4** is a diagram which shows an effect of the present invention, showing growth suppression on leukemia cell lines (HL-60 lines) in the presence of an agaricus extract (ABMK-22);
Figure **5** is a diagram which shows an effect of the present invention, showing growth suppression on leukemia cell lines (HL-60lines) in the presence of an agaricus extract (ABMK-22);
Figure **6** is a diagram which shows an effect of the present invention, showing growth suppression on leukemia cell lines ( HL-60 lines) in the presence of an agaricus extract (ABMK-22);
Figure **7** is a diagram which shows an effect of the present invention, showing differentiation induction on leukemia cell lines (HL-60 lines) in the presence of an agaricus extract (ABMK-22);
Figure **8** is a diagram showing differentiation induction of a differentiation induction agent (ATRA) on leukemia cell lines (HL-60 lines);
Figure **9** is a diagram which shows an effect of the present invention, showing apoptosis induction of an agaricus extract (ABMK-22) on leukemia cell lines (HL-60 lines);
Figure **10** is a diagram which shows an effect of the present invention, showing apoptosis induction of an agaricus extract (ABMK-22) and differentiation induction agent (tretinoin) when used together on leukemia cell lines (HL-60 lines);
Figure **11** is a diagram showing differentiation induction of a differentiation induction agent (VD₃) on leukemia cell lines (HL-60 lines);
Figure **12** is a diagram showing growth suppression of an ingredient included in an agaricus extract (ABMK-22) on leukemia cell lines (HL-60 lines);
Figure **13** is a diagram showing apoptosis induction of an ingredient included in an agaricus extract (ABMK-22) on leukemia cell lines (HL-60 lines);
Figure **14** shows an immune activation activity of an ingredient included in an agaricus extract (ABMK-22);
Figure **15** shows an immune activation activity of an ingredient included in an agaricus extract (ABMK-22); and
Figure **16** shows an immune activation activity of Picibanil.

### BEST MODE FOR CARRYING OUT THE INVENTION

Hereinafter a material derived from agaricus which induces apoptosis of cells or a method for producing a composition according to the present invention will be described.

The agaricus used in the present invention is a fruiting body or mycelium portion of natural or artificially-cultured agaricus. For convenience, commercially available dried fruiting bodies can also be used. The agaricus is used as it is, is cut, or is powdered and provided for preparing an agaricus extract.

The agaricus extract is an extract of agaricus including an agaricus-derived ingredient obtained by using water, lower alcohols, or the like as a solvent. Typically, water, ethanol, aqueous ethanol, or the like is used. Any solvent can be used as long as a fraction having an activity of inducing apoptosis of cells can be extracted. In general, the dried fruiting body, or the powder thereof, is mixed with a solvent of 2 through 10 times the weight thereof to perform extraction. Examples of solvents include water, ethanol, propanol, butanol, acetone, 1,3-butylene glycol, ethyl acetate, hexane, methylene chloride, methanol, or a mixture thereof.

Typically, the agaricus extract can be obtained by using water as a solvent. It is prepared by extraction with hot water from agaricus.

Typically, extraction from agaricus with hot water is performed by mixing dried fruiting body/bodies with 5 through 10 times the weight thereof of water, and heat-refluxing the mixture for 1 through 3 hours. Hot water extraction from agaricus may be performed by repeating the heat-reflux on a residue previously extracted with hot water. The solution extracted with hot water thus obtained is dried by a method known to those skilled in the art such as lyophilization, spray-drying, or the like to obtain a dried product (hereinafter, referred to as dried product A). Dried product A is mixed with 5 through 20 times the weight thereof of water. Then, the solution is put into a dialysis tube and dialyzed for 10 through 15 hours with several times the amount thereof of distilled water. The obtained dialyzate is lyophilized to obtain a dried product (hereinafter, referred to as dried product C) having an activity of inducing apoptosis of cells.

Then, the solution remaining in the dialysis tube is further dialyzed against running water for 20 through 40 hours and dialyzed twice against distilled water for a few hours each time and a dried product of the solution remaining in the dialysis tube is obtained as described above. Thus, the dried product (hereinafter, referred to as dried product B) having an activity of inducing apoptosis of cells can be obtained.

Next, obtained dried product C is dissolved into about ten times the weight thereof of distilled water. Chromatography is performed with distilled water as an eluent to obtain 20 mL fractions. From the obtained fractions, a main ingredient in the middle fractions which has a molecular weight of about 100-2000 Da by gel filtration is an ingredient having the activity of inducing apoptosis of cells, according to the present invention.

These fractions are analyzed further using reverse-phase chromatography which uses ODS (octadecyl silanated silica gel), ion-exchange chromatography using DEAE-TOYOPEARL 650, or the like, and confirmed to include a plurality of ingredient s such as arginine, lysine, mannitol, and the like.

The solution extracted with hot water, obtained by the above-described method, is mixed with an equal amount of ethanol. The mixture is centrifuged to separate precipitate and supernatant. The obtained supernatant is further mixed with ethanol of 1 through 3 volumes thereof. The mixture is further centrifuged to obtain precipitate. The precipitate obtained is dissolved in distilled water and the solution obtained is dialyzed. The dialyzate obtained is also a low-molecular weight fraction, which has the activity of inducing apoptosis of cells, according to the present invention.

The agaricus extract obtained as described above or fractionsthereof, whichhavetheactivitytoinduceapoptosis of cells, canbe used for production of medicines by themselves or in combination with various carriers used for producing an orally taken medicine. Further, the extract of agaricus with hot water or the fractions thereof, which show the activity of inducing apoptosis of cells, may be used as health foods by themselves or by using with other foods.

Typically, the composition of the claimed invention can be taken orally with a biocompatible pharmaceutical carrier (for example, physiological saline, buffered physiological saline, dextrose, water, or the like). The compositions of the present invention can be taken by itself or in combination with other medicines or food materials.

The compositions of the present invention can be administered orally or parenterally. Parenteral administration includes topical, dermal, intra-arterial, intramuscular, subcutaneous, intramedullary, intra-cisternal, intraventricular, intravenous, intra-abdominal, or intranasal administration. Preferably, the compositions of the present invention are administered by intravenous or intra-arterial injection. The details of formulation and administration of the pharmaceutical composition according to the present invention can be performed in accordance with descriptions in a textbook in the field of art, for example, "REMINGTON'S PHARMACEUTICAL SCIENCES" (Maack Publishing Co., Easton, PA).

A composition for oral administration can be formulated as a composition including a pharmaceutically acceptable carrier well known in the art in a prescription form suitable for administration. Such a carrier allows the composition obtained to be formulated as a tablet, pill, sugar-coated pill, capsule, liquid, gel, syrup, slurry, suspension, or the like, suitable for ingestion by patients.

The composition of the present invention includes the agaricus extract in an amount effective for inducing apoptosis. Those skilled in the art will thoroughly understand and recognize the "pharmacologically effective amount". The term "pharmacologically effective amount" can be sufficiently recognized by those skilled in the art, and refers to an amount of agaricus extract which is effective for alleviating intended cancer symptoms. Thus, the pharmacologically effective amount is an amount sufficient for inducing apoptosis.

An example of assays useful for confirming the "pharmacologically effective amount" is to measure a degree of alleviation in cancer symptoms in a subject. The amount of the agaricus extract which is actually administered depends on the health conditions of the individual to which the extract is applied and may be optimized so that a desirable effect can be achieved. It is a routine process for those skilled in the art to determine a pharmaceutically effective amount.

The pharmacologically effective amount can be first evaluated by *in vitro* assay using cell culture or suitable animal models. Then, based on such information, an amount and a route which are effective in administration to a human can be determined.

As a guideline, and without limitation, an amount sufficient for inducing apoptosis is 0.1-30.0 g for a person per day, and preferably 3-15 g for a person per day, when administered to an adult as a dried solid of the fraction inducing apoptosis according to the present invention.

The agaricus extract or a fraction thereof having the activity of inducing apoptosis can be mixed with one or more selected food materials in an amount sufficient for exerting its function. The one or more selected food materials are mixed with the extract having immune activation activity in a form known to those skilled in the art, usually, powder form. The mixture can be served as a liquid food product depending on its utility or on preference. Alternatively, the mixture may be formed as capsules such as hard capsules or soft capsules, tablets, or pills, or may be formed into a powdery, granular, tea-leaf, tea-bag, or candy form.

Hereinafter, the present invention will be further described by way of examples. The following examples are merely illustrative and do not limit the present invention.

### Example

### (Example 1)

2 L of distilled water was added to 300 g of Kyowa's *Agaricus blazei* Murill mushroom, and the mixture was heated to reflux for two hours. The solution obtained was filtered to separate a filtrate (a solution extracted with hot water) and a residue. Again, 2 L of distilled water was added to the residue and the mixture was heated to reflux for another two hours to perform hot water extraction and a filtrate was obtained. Further, the same procedure was repeated one more time. The filtrates obtained were lyophilized together to obtain dried product A (153 g: extraction rate (recovery) of 51%).

500 mL of distilled water was added to 50 g of dried product A and the mixture was put into a dialysis tube (Spectra/Por Membrane 50x31, inner diameter of 8 mm and length of 30 cm, FE-0526-65). The mixture was dialyzed against 3 L of distilled water for 12 hours. The dialyzate obtained was lyophilized to obtain dried product C (27 g: extraction rate of 53%). The solution remaining in the dialysis tube was further dialyzed against running water for 30 hours, and then dialyzed twice against distilled water (four hours each time, total 8 hours). Thereafter, the solution remaining in the dialysis tube was lyophilized to obtain dried product B (11 g: extraction rate of 22%). Subsequently, 3 g of dried product C was dissolved in 30 mL of distilled water and chromatography using TOYOPEARL HW40C (inner diameter of 40 mm and length of 420 mm) was performed. The eluent was entirely distilled water. For each fraction, 20 ml of the aliquots were taken to obtain fractions 1 through 30. These fractions were divided into the following five groups with reference to results of thin-layer chromatography analysis. The dried weights were as follows: fractions 1 through 11 (75 mg, 2.5%); fractions 12 through 15 (920 mg, 30.7%); fractions 16 through 17 (1570 mg, 52.3%); fractions 18 through 19 (270 mg, 9%); and fractions 20 through 28 (97 mg, 3.2%).

Infrared radiation (IR) absorption spectrum data of fraction 16 (hereinafter, referred to as 1SY-16) was as follows.
Fraction 16: IR (KBr) 3390, 3325, 3285, 2940, 2920, 1641, 1634, 1622, 1615, 1600, 1595, 1405, 1394, 1084, 1020: molecular weight (estimated by gel filtration) 100-2000 Da

### (Example 2)

Hot water extraction similar to as described in Example 1 was performed to obtain 6 L of a combined filtrate (a solution extracted with hot water). The filtrate was concentrated under reduced pressure to 1 L, and 1 L of ethanol was added thereto and mixed to separate polysaccharides. The mixture was centrifuged to obtain precipitate and supernatant. 3 L of ethanol was further added to the supernatant and mixed, and the mixture was centrifuged to obtain a precipitate, and the precipitate was dissolved in distilled water and dialyzed. The dialyzate obtained was lyophilized to obtain a powder referred to as ABMK-22.

### (Example 3)

The lyophilized powder of the dialyzate of the agaricus hot water extract (ABMK-22) was examined with respect to cell growth suppression activity (Test 1), cell differentiation inducing activity (Test 2), and apoptosis inducing activity (Test 3).

The bioactivity tests were performed by the following methods.

### 1. Cell growth suppression activity (Test 1): For assay, the Collagen gel droplet embedded culture-drug sensitivity test (CD-DST method) and a counting chamber were used.

The CD-DST method was performed by following the method of H.Kobayashi et al. (INTERNATIONAL JOURNAL OF ONCOLOGY 11:449-455,1997).

In brief, the method is as follows: First, a suspension of subject cells (HL60 cell line) was mixed with collagen (for example, Type I collagen (Cellmatrix Type CD, Nitta Gelatin Inc.)), reconstitution buffer, and a culture medium (for example, concentrated F-12 medium) in ice water . to embed subject cells in a collagen gel. The resultant mixture was placed in wells of a multi-well plate such that 10³ to 10⁴ cells were placed per well (which requires about three drops of collagen gel droplets), and was cultured in a CO₂ incubator at 37°C overnight. Then, the subject drug was added at a predetermined concentration and cultured for 24 hours. The drug was then removed, and cells in the wells were rinsed with a buf f er, overlaid with medium, and incubated for a further 7 days. After culturing, colonies grown in the collagen gel were stained with neutral red. Eachcollagen droplet was fixed with 10% neutral formalin buffer, washed in water, air dried, and subjected to an image analysis. The cell growth suppression effect was obtained by calculating a complementary bulking value of only the cancer cell colonies using growth morphology of cancer cells, and a difference in extent of neutral red staining to obtain a ratio of relative growth rate between a group which was not treated with ABMK-22 and a group which was treated. The CD-DST method has been developed as a drug sensitivity test method for solid cancers. However, since the method is a growth assay method, it can be applied for measuring a state of growth of cells.

A method using a counting chamber was performed following the method described in Rinsho Kensaho Teiyo (Kanai's Manual of Clinical Laboratory Medicine), 30th Ed. (August 20, 1993; Kanehara & Co., Ltd.; Tokyo).

### 2. Cell differentiation inducing activity (Test 2): Evaluation was made using Nitro blue tetrazolium (NBT) reduction power.

NBT reduction was performed in accordance with the method of S. J. Collins et al. (Int. J. Cancer: 25, pages 213-218 (1980)). In brief, the method is as follows: about 2×10⁵ HL60 cells were pelleted, and washed in RPMI-1640 medium. Then, the cells were suspended in RPMI-1640 medium containing 0.1% NBT and 100 ng/ml of phorbol ester (TPA), and cultured for forty minutes at 37°C. The resultant culture was observed under microscope and cells containing blue-black deposits were assessed as differentiated cells.

### 3. Apoptosis inducing activity (Test 3): Measurement was performed using a terminal deoxynucleotidyl transferase(TdT)-mediated deoxyuridine triphosphate (dUTP)-biotin nick end-labeling method (TUNEL method). The TUNEL method is a method for identifying apoptosis on a tissue slice reported by Gavrieli et al. in 1992 (Yael Gavrieli et al., The Journal of Cell Biology, vol. 119, No.3, November, 1992, p. 493-501). The method is known to be a method which enables observation of apoptosis at the individual cell level at various states of morbidity and allows comparison with morphology changes. The TUNEL method was performed with HL-60 cell lines using Apoptosis in situ Detection Kit wako (Wako Pure Chemical Industries, Ltd.), according to the method of Gavrieli et al., supra.

### 4. Measurement results of cell growth suppression activity (Test 1)

Based on the results of measurement using HL60 cell lines, it became clear that the CD-DST method is not suitable for measuring the growth state of suspended cells. Therefore, the cell growth suppression activity was measured using a counting chamber.

First, growth of HL60 cell lines was examined in the presence of ABMK-22 at various concentrations to obtain a growth curve of the HL60 line. More specifically, an initial number of cells of HL 60 cell lines was set to be 200,000/mL. ABMK-22 was added to have concentrations of 0.1, 0.25, 0.5, 0.75, 1, 2, 2.5, 3, 4 and 5 mg/1 mL, respectively and cultured. The number of the cells was measured over time for four days.

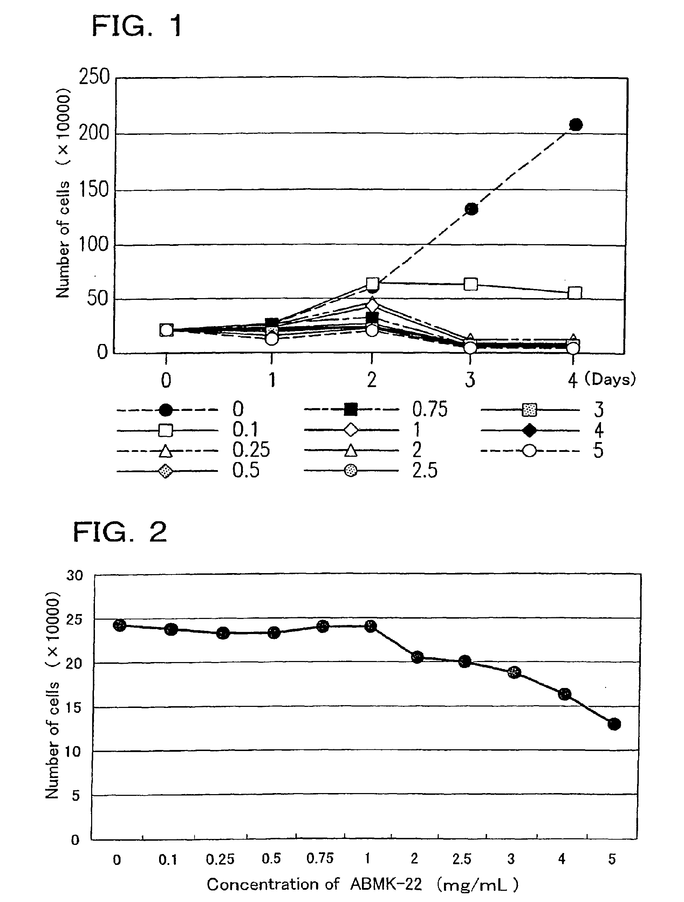

Figure **1** shows a curve indicating growth of HL60 cells in the presence of ABMK-22 at each of the concentrations. Each of the symbols in Figure **1** indicates the result of measurement of the number of the cells (number/ml) in the presence of ABMK-22 at the concentration of the number indicated in the lower part of Figure **1** by the symbols. Figures **2** through **5** show the results of measurement of the number of cells at the first day, second day, third day, and fourth day of culture which are plotted with respect to the concentration of the ABMK-22 for each of the four days of culture.

As shown in Figure **2**, on the first day of the culture, ABMK-22 at concentrations up to 1 mg/mL did not affect the growth HL60 cells. ABMK-22 at the concentration of 2 mg/mL or higher suppressed the growth of HL 60 cells in a concentration-dependent manner.

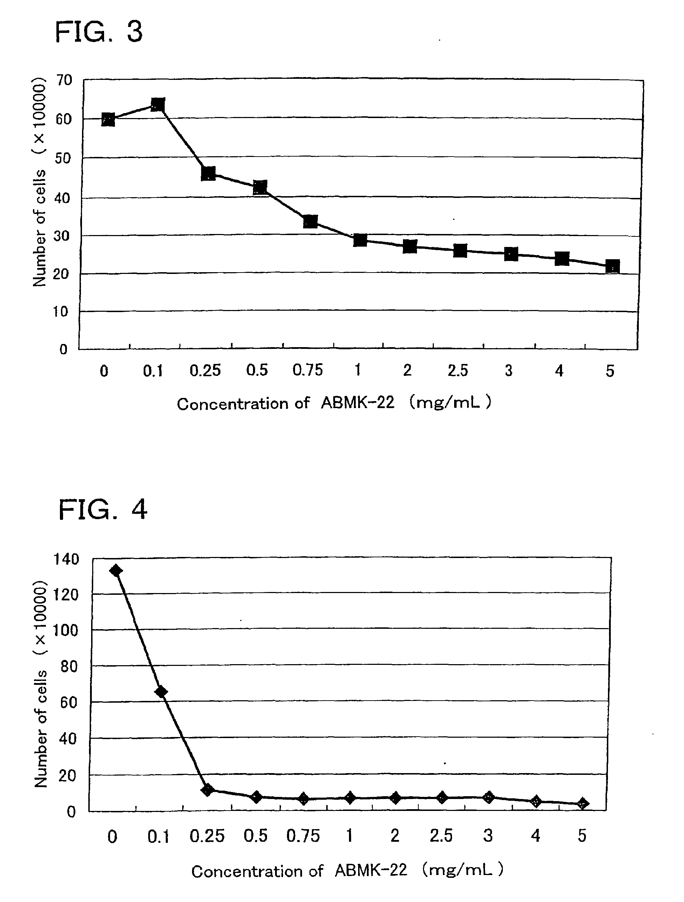

As shown in Figure **3**, on the second day of the culture, ABMK-22 at the concentration of 0.25 mg/mL or higher suppressed the growth of HL 60 cells compared to the control in a concentration-dependent manner.

As shown in Figure **4**, on the third day of the culture, ABMK-22 at the concentration of 0.1 mg/mL suppressed about 50% of the growth of HL 60 cells compared to the control. The cells whose growth was suppressed also experienced cell death observed morphologically. In the presence of the ABMK-22 at the concentration of 0.25 mg/mL or higher, the number of the cells that were alive are remarkably decreased. A superior cell-killing effect was shown.

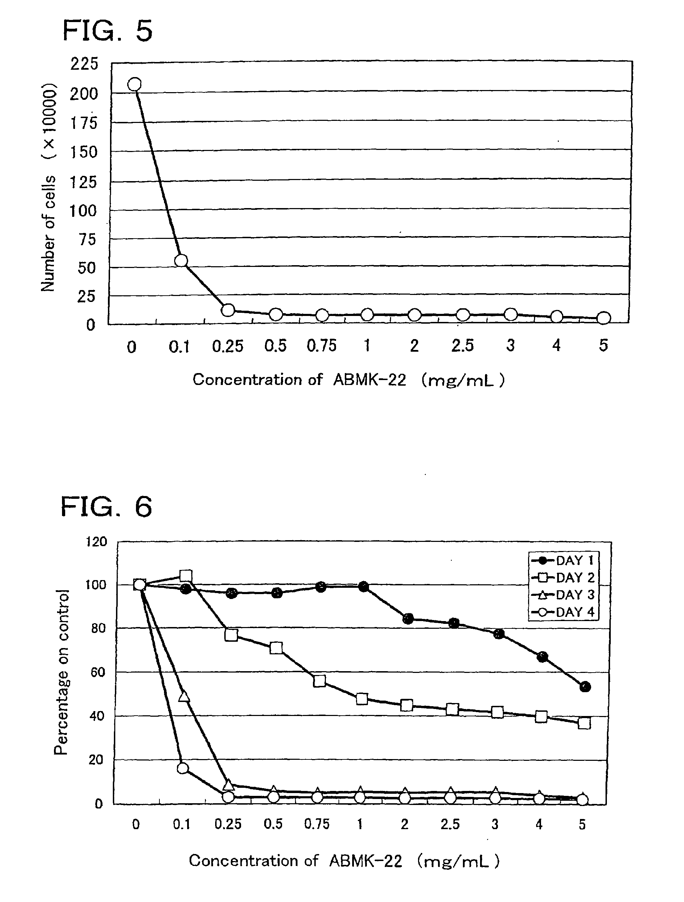

As shown in Figure **5**, on the fourth day of the culture, ABMK-22 at the concentration of 0.1 mg/mL suppressed about 84% of the growth of HL 60 cells compared to the control. The cells whose growth was suppressed also experienced cell death observed morphologically. As in the third day of culture, the ABMK-22 at the concentration of 0.25 mg/mL or higher, shows a superior cell-killing effect against the HL60 cells.

Figure 6 shows the results above as an effect of growth suppression on control (T/C%) over time. The vertical axis represents the growth suppression effect on control (T/C%), and the horizontal axis represents the concentration of the ABMK-22 added to the culture. As shown in Figure **6**, ABMK-22 shows the growth suppression effect on HL60 cells in a time-dependent manner. Specifically, the suppression effect on HL60 cells in a concentration-dependent manner were observed at the concentration of 2 mg/mL or higher on the first day of the culture (filled circles), and at the concentration of 1 mg/mL or higher on the second day of the culture (unfilled squares). A superior growth suppression effect on HL60 cells was observed at a low ABMK-22 concentration on the third day (unfilled triangles) and the fourth day (unfilled circles) of culture. This suppression effect was confirmed as being to due to cell death.

### 5. Measurement results of cell differentiation inducing activity (Test 2)

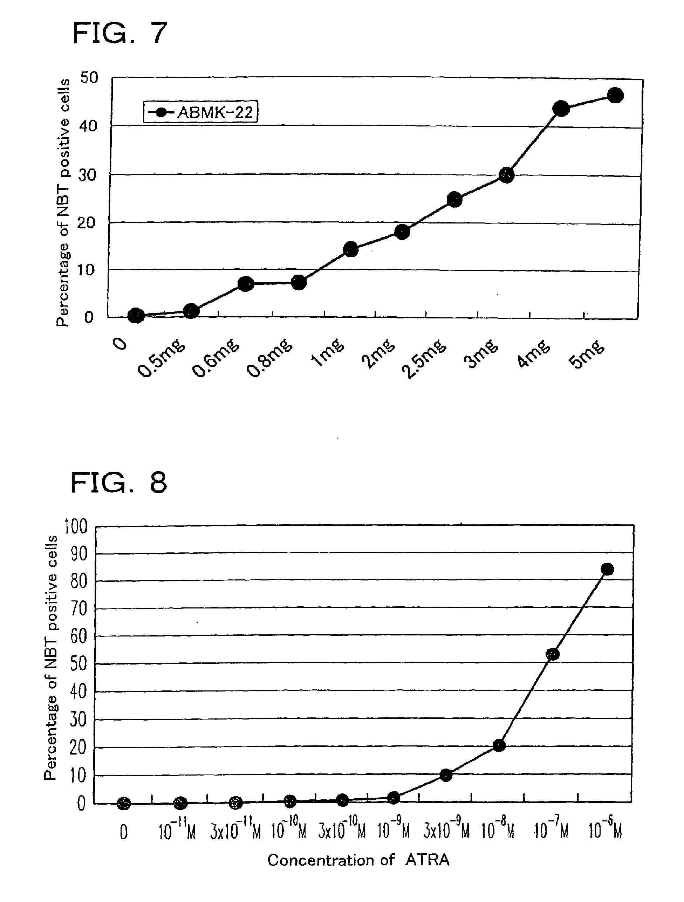

In the presence of ABMK-22 at various concentrations , HL60 cells were cultured for four days and the differentiation inducing power of ABMK-22 was measured. The results of measuring NBT reducing power, which is an indicator of differentiation of a HL60 cell line under each concentration, are shown in Figure **7**. In Figure **7**, ABMK-22 increases NBT positive cells in a concentration-dependent manner. At a concentration of 4 mg/mL, about 33% of the cells that were alive were differentiated. This value indicates a superior induction activity corresponding to 10⁻⁸ to 10⁻⁷ M of all-trans retinoic acid (ATRA) used as control of differentiation of HL60 cell lines (see Figure **8**). Figure **8** shows the results of measuring the cell differentiation induction activity of ATRA in the same assay system.

### 6. Measurement results of apoptosis induction activity (Test 3)

As indicated by the above results, ABMK-22 exhibited cell-killing effects in a time-dependent manner even at a low concentration. It is considered that ABMK-22 induces apoptosis. Thus, the apoptosis induction activity of ABMK-22 was confirmed by the TUNEL method.

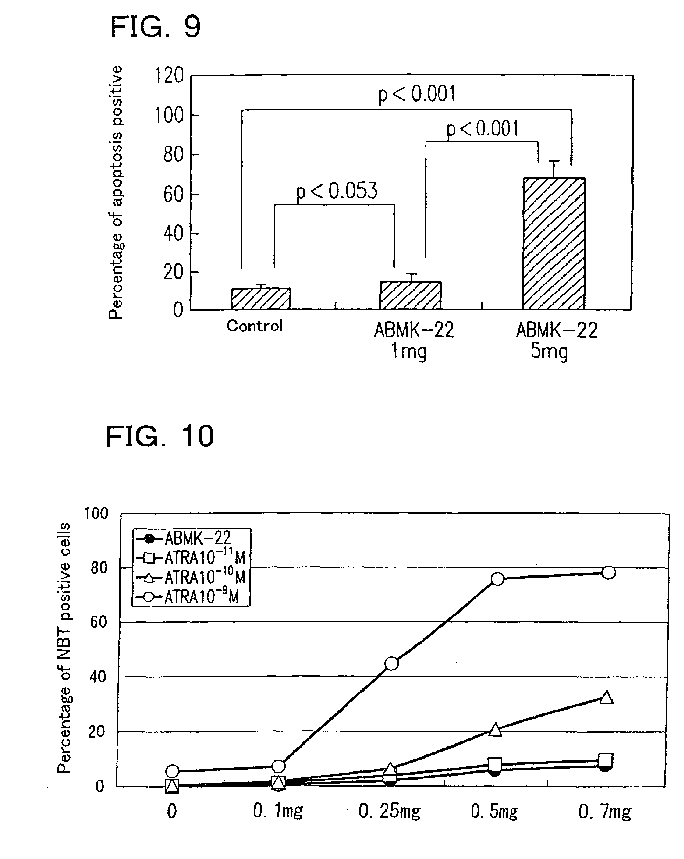

Based on the above results of adding ABMK-22 for one day (shown in Figure **2**), the concentration of 1 mg/mL was selected as it is the concentration at which cell growth is the same as that in the control and the concentration of 5 mg/mL was selected as it is the concentration at which the growth was remarkably reduced. ABMK-22 was added for one day and the activity of inducing apoptosis was measured. The results are shown in Figure **9**. The results of Figure **9** show results measured for respective test groups. As shown in Figure **9**, when ABMK-22 was added at a concentration of 1 mg/mL, the tendency of apoptosis being induced compared to the control exhibited p < 0.053. At a concentration of 5 mg/mL, apoptosis was induced significantly (p < 0.001). When concentrations of 1 mg/mL and 5 mg/mL were compared, apoptosis was induced significantly at the concentration of 5 mg/mL (p < 0.001) (Student t- test). Based on the above results, cell death of HL60 cell lines byABMK-22 are confirmed to be death caused by the induction of apoptosis.

### (Example 4) Effect of using ABMK-22 along with a differentiation induction agent

As a substance having a differentiation inducing effect on HL60 cell lines, an active-type vitamin D₃ formulation (VD₃) described above and all-trans retinoic acid (ATRA; tretinoin) are known.

HL60 cell lines are induced to differentiate into monocyte and macrophage lines by VD₃ and granulocyte lines by ATRA. Figure **10** shows the results of evaluating cell differentiation induction activity of the HL60 cell lines by NBT reducing power in combination with ABMK-22 at various concentrations and ATRA at concentrations of 10⁻⁹ to 10⁻¹¹ M. As shown in Figure **10,** when ABMK-22 is used together with a low concentration ATRA (10⁻⁹ M through 10⁻¹¹ M), differentiation was induced in a concentration-dependent manner, and when ABMK-22 was used together with ATRA at concentration of 10⁻⁹ M, differentiation was significantly enhanced, and the effect of using both together was recognized.

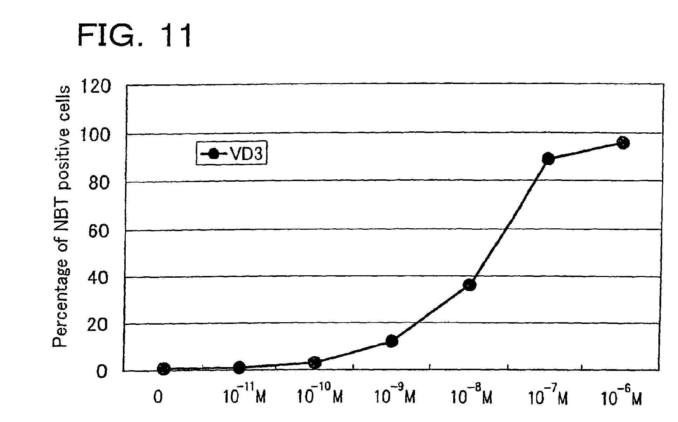

Figure **11** shows the results of evaluating the cell differentiation induction activity of HL60 cell lines by NBT reducing power when 1α, 25(OH)2D3 (active-type vitamin D₃, hereinafter referred to as VD₃) at the concentrations of 10⁻⁶ M to 10⁻¹¹ M was used by itself. As shown in the figure, when VD₃ concentration is 10⁻¹⁰ M or lower, VD₃ by itself does not exhibit cell differentiation induction activity. Further, as shown in Figure **8**, when the concentration of ATRA is 10⁻⁹ M or lower, ATRA does not exhibit differentiation induction activity.

It is considered that growth, differentiation and apoptosis of cancer cells are related to each other. Suppression of growth by a certain substance stops a cell cycle. After the suppression of growth, differentiation is induced. Apoptosis directly induces signals for death. It is considered that growth, differentiation and apoptosis of cells have differences only in the switches for deciding direction. Therefore, a difference of a certain substance in quality and quantitymayprobably induce growth suppression, differentiation, or apoptosis of cells simultaneously.

Regarding differentiation induction of cancer cells, VD₃ or ATRA have already been used clinically. However, due to side effects (for VD₃, hypercalcemia, andforATRA, retinoic acid syndromes), VD₃ is mainly used as a medicine for external use, and ATRA is only used for APL (promyelocytic leukemia: a cancer in which gene mutations are evident).

The results shown herein, particularly, the results shown in Figures **8** and **10**, indicate that differentiation can be significantly induced, under conditions of low concentration in which the differentiation induction effect of the differentiation inducing substance is not expressed, by using the substance in combination with other substances (particularly, agaricus). Further, these results show that the differentiation may be significantly induced by also combination of ABMK-22 and vitamin A-like substance (carotenoid). The present inventors actually obtained results proving this.

### (Example 5) Apoptosis induction activity of an ingredient included in ABMK-22

For identifying the ingredient having the apoptosis induction activity in ABMK-22 obtained in Example 2, ABMK-22 was further purified and fractionated. The obtained ingredient was subjected to the method described in section "3. Apoptosis inducing activity (Test 3)" in Example 3 above to examine the apoptosis induction activity of the obtained ingredient.

### 1. Purification and fraction of the ingredients included in ABMK-22 and structural analysis

ABMK-22 obtained by the method described in Example 2 (about 13.5 mg) was dissolved in 30 mL of distilled water. Reverse-phase chromatography was performed using ODS (50 mm idx300 nm) as a carrier. Solvents used for elution were distilled water, 5% methanol/water, 70% methanol/water and 100% methanol. By using these solvents, ingredients were eluted in order. For each of the fractions, 200 mL aliquots were taken. With reference to the results of analysis by thin-layer chromatography, each of the fractions were separated into the following five groups: ABMK2201 (18.7g, 93.5%); ABMK2202 (445 mg, 2.2%); ABMK2203 (36.9 mg, 0.18%); ABMK2204 (3.5%); and ABMK2205 (91.3 mg, 0.46%). Then, ABMK2202 was applied to high-performance liquid chromatography (HPLC, ODS column 20 mm idx250 mm) 50 mg at a time and eluted with 5% methanol/water. Thus, ABMK6873 (34.7 mg, 0.17%) and ABMK0415(2.2 mg, 0.011%) were isolated, respectively.

ABMK6873 and ABMK0415 were subjected to mass spectrometry and NMR analysis at the following conditions. As a result of analyzing the results, the two ingredients were confirmed to be β-N-(γ-glutamyl)-4-formyl phenylhydrazine) (having a structure represented by the following formula I) and N-(3-carboxypropyl)-2-formyl-5-hydroxymethylpyrrole (having a structure represented by the following formula II).

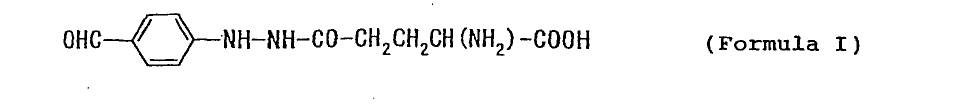

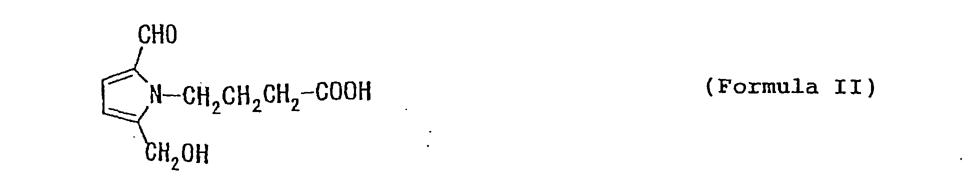

### [Mass spectrometry]

The lyophilized powder from the obtained peak ingredient was mixed with milli-Q water so as to provide an aqueous solution of the concentration of 10 mg/ml analyte. As a measurement device, a JEOL HX110/110A tandem type mass spectrometer was used. In mass spectrometry methods FAB, EI, CI, and HRFAB, measurements were performed in positive measurement mode (FAB, EI, CI), negative measurement mode FAB, CI), and negative measurement mode (HRFAB). Resolution power in mass spectrometry was 1000 for FAB, EI, and CI, and 5000 for HRFAB.

### (1) FAB-MS

Glycerol was used as matrix. Glycerol and sample were mixed at ratio of 1:1 (v/v) on a sample mounting stage, and then measured immediately. For mass calibration, a mixture of alkali ion was used in the positive mode and a glycerol solution of cesium iodide (CsI) was used in the negative mode.

### (2) EI-MS and CI-MS

The sample itself was introduced into an ion source. For CI, isobutane was used as ion gas.

### (3) HRFAB-MS

PEG-500 was used as amass calibration sample. After glycerol and the sample were mixed at ratio of 1:1 (v/v) on a sample mounting stage, an appropriate amount of PEG-500 was added, and then measured in the negative mode immediately. Mass calibration was performed with two peaks of PEG-500 sandwiching a target peak ([M-H]⁻=210) selected.

### [NMR analysis]

NMR analysis was performed using a Bruker DMX500 nuclear magnetic resonance apparatus (¹H500MHz) and a JEOL JNM-A400 nuclear magnetic resonance apparatus (¹H400MHz). After mass spectrometry, the samples were lyophilized and dissolved in heavy water (0.3 ml) and provided for measurement. Further, the sample with the solvent again replaced with heavy chloroform was also analyzed.

### [Results of analysis]

### ABMK0415:

In all spectra in the above described mass spectrometry modes, peaks corresponding to a molecular weight of 211 were observed. Thus, the molecular weight of the compound was determined to be 211. Then, HRFAB-MS measurement was performed in the negative mode having better peak sensitivity, the observed precise mass (m/z=210.0757) shows good match with C₁₀H₁₂O₄N (-1.0 mmu). Thus, molecular formula of the peak I ingredient was determined to be C₁₀H₁₈O₄N.

Next, NMR measurements were performed in order to obtain structural information. As a result of analysis based on cross peak information of HMBC spectrum, the structure represented by formula II was estimated.

### ABMK6873:

In FAB-MS, a pseudo-molecular ion MH+ were observed with m/z 266. In EI-MS, M⁺ ions were observed with m/z 649(C₂₀H₁₁F₁₂N₃O₃), and [M-H₂O]⁺ ions were observed with m/z 331 for trifluoroacetic acid and acetic acid derivatives. By H¹NMR, aldehyde proton, p-substituted aromatic ring and glutamic acid residue -CO-CH₂-CH₂-CH(NH₂)-COOH were characterized. Deshielding of two aromatic protons (d7.91ppm), UV absorption in neutral solution (313 nm) and alkaline solution (385 nm), and important generation of ion at m/z 136(C₇H₈N₂O) match those for a p-formyl phenylhydrazine unit.

UVλ¹²⁰ₘₐₓnm(logε); 231(3.79), 313(4.19); +NaOH 248(3.76), 385(4.30); +HCl 231(3.79), 313(4.19): FAB⁺MS m/z(relative strength): 266(MH⁺, 100), 201(62), 166(16), 137(20), 136(15), 132(39), 121(16); ¹H NMR(400 MHz, D₂O) 9.73(1H, s, -CHO), 7.91(2H, d, J=8.5Hz), 7.03(2H, d, J=8.5Hz), 3.91(1H, t, J=6Hz, Hα), 2.67 (2H, m, Hγ), 232(2H,m,Hβ)

### 2. Apoptosis induction activity of the ingredient included in ABMK-22

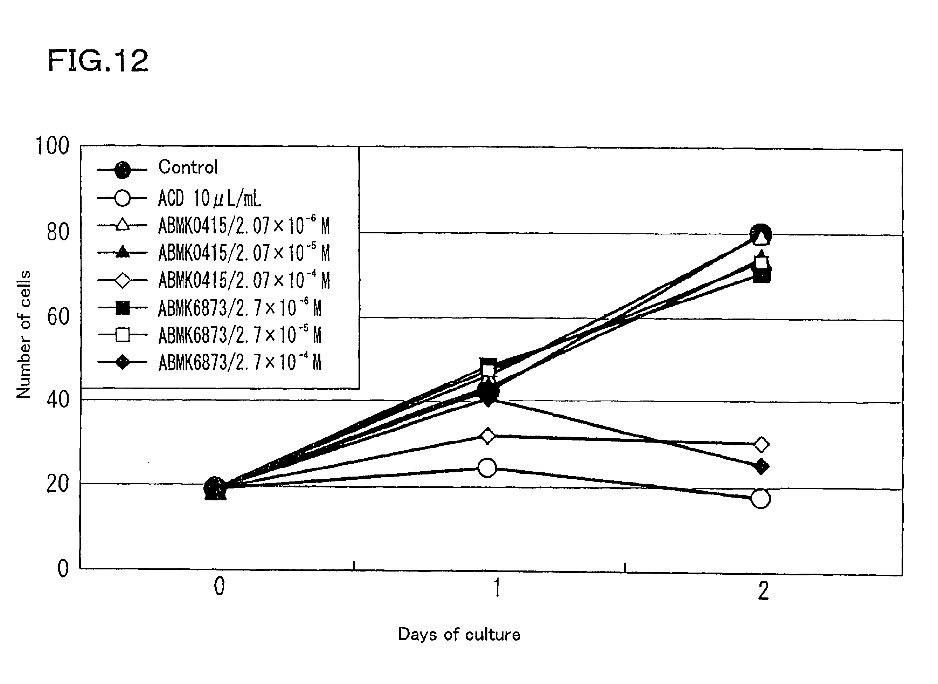

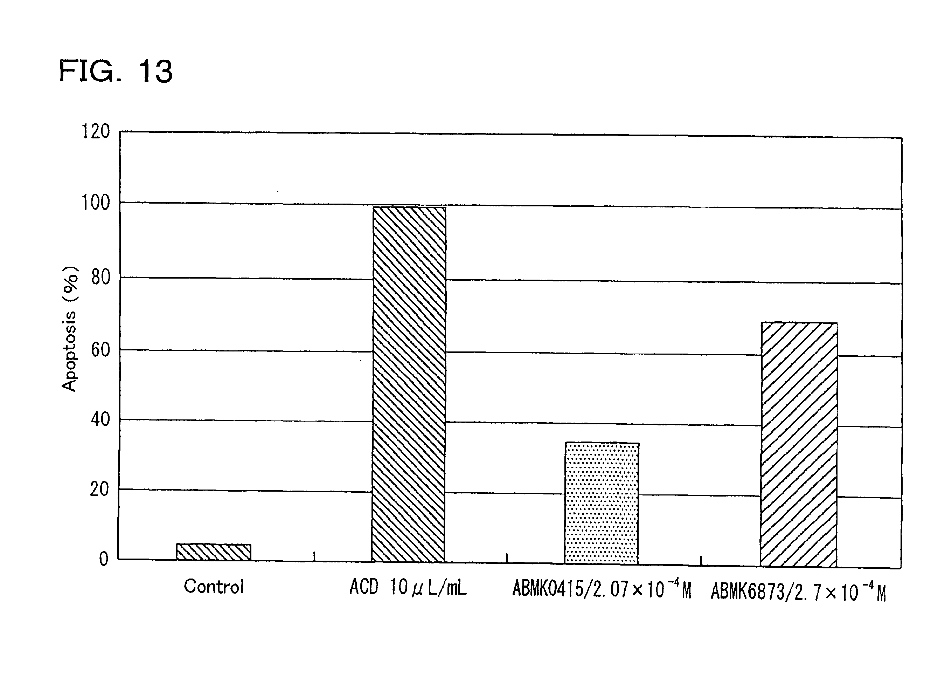

Next, the apoptosis induction activity of a lyophilized powder of ABMK0415 and ABMK6873 were measured as described in section "3. Apoptosis inducing activity (Test 3) " in Example 3 above. ABMK0415 was tested at concentrations in the range of 2.07×10⁻⁶M to 2.07×10⁻⁴M. ABMK6873 was tested at concentrations in the range of 2.7×10⁻⁶M to 2.7×10⁻⁴M. The results are shown in Figures 12 and 13.

Figure **12** shows a growth curve of HL-60 cell lines in the presence of ABMK0415 and ABMK6873 at various concentrations. Symbols shown in Figure 12 show the results of the measurement of the number of cells in the presence (number/ml) of ingredients at the concentration as specified by the corresponding symbols in the upper-left portion of Figure **12**. Unfilled circles indicate the results for actinomycin D (ACD) of positive control and filled circles indicate the measurement results for the control sample group without drug.

As shown in Figure **12**, both ABMK0415 and ABMK6873 suppress growth of HL-60 cells in nearly a concentration-dependent manner. Particularly, it is demonstrated that, in the presence of ABMK0415 at the concentration of 2.07×10⁻⁴M and ABMK6873 at the concentration of 2.7×10⁻⁴M, the growth of HL-60 cells are remarkably suppressed.

Figure **13** shows the percentage of apoptosis positive cells in the presence of ABMK0415 at the concentration of 2.07×10⁻⁴M and ABMK6873 at the concentration of 2.7×10⁻⁴M after culture for two days. In Figure **13**, the horizontal axis indicates each sample group, and the vertical axis indicates the rate of the cells which experience apoptosis. As shown in Figure 13, in the presence of ABMK0415 at the concentration of 2.07×10⁻⁴M, apoptosis was induced in about 35% of the cells, and in the presence of ABMK6873 at the concentration of 2.7×10⁻⁴M, apoptosis was induced in about 70% of the cells. ABMK0415 and ABMK6873 were confirmed to have apoptosis induction power.

### (Example 6) Measurement of immune activation activity of the ingredient included in ABMK-22

The immune activation activity of ABMK0415 and ABMK6873 were measured.

Dendritic cells are involved in the establishment of immunity. Dendritic cells may also be called arborescent cells, dendritic leukocyte, or D cells. This is the generic term for cell groups having dendritic form derived from myelocytes distributed in various tissue organs in the living body except for the brain. The cell groups include lymphoid dendritic cells, Langerhans cells, veil cells, interconnected cells, and interstitional cells. Accompanying an inflammation response caused by invasion of foreign matter, the cells perform pinocytosis, and move from a local spot via the vas afferens to an associated lymph node or to the spleen through bloodstream. The cells are known to start an immune response after they reach a T cell-dependent region by presenting an antigen in a form combined with class II antigen and by activating specific T cells. Namely, the cells play an important role in the immune system. In the present example, the immune activation activity of ABMK0415 and ABMK6873 were confirmed by assaying the induction power of monocytes to dendritic cells.

### 1. Materials and method

### 1.1. Materials

Peripheral blood of two healthy people was used as a material with donor consent.

### 1.2. Separation of peripheral blood mononuclear cells (PBMC)

PBMC were separated from a whole blood specimen which was collected with heparinization by a Ficoll-Paque density centrifugal separation method.

### 1.3. Test samples

ABMK0415 and ABMK6873 were used as test samples. ABMK0415 and ABMK6873 were respectively dissolved in culture media of cells used for tests (10% FCS added RPMI-1640, 5% human AB serum added RPMI-1640) such that each mixture has a concentration of 200 mg/ml. The mixtures were filter-sterilized through a filter having a pore diameter of 0.22 µm to obtain test samples.

### 1.4 Measurement of immune activation activity

An effect of immune activation of novel compounds was examined *in vitro* through induction power for dendric cells (DC) derived from human peripheral blood monocytes.

PBMC was processed with a dish treatment for one hour with a carbon dioxide gas incubator. Thus, a monocyte-rich attachment cell fraction was obtained. The cells obtained were cultured in 5% human AB serum added RPMI-1640 (NIKKEN BIOLOGICAL MEDICAL RESEARCH), in the presence of 500 U/ml of GM-CSF (DIACLONE Research) and IL-4(BD Biosciences).

For culturing, a dish (NUNC) having a diameter of 3. 5 cm was used. On the sixth day of culture, Picibanil and the novel compound were added such that the final concentration is 0.1 KE/ml for Picibanil, and 200 µg/ml for ABMK0415 and ABMK6873. Picibanil (Chugai Pharmaceutical Co. , Ltd.) was used as a positive control which has a DC induction power. The negative control was cells cultured similarly by using 5% human AB serum with added RPMI-1640.

All DCs in the dish were collected on the seventh day after the culture started. The surface antigen thereof was analyzed by flow cytometry using a monoclonal antibody (anti-CD80; BD Bioscience). A significant-difference test was performed using the Student's t-test.

### 2. Results

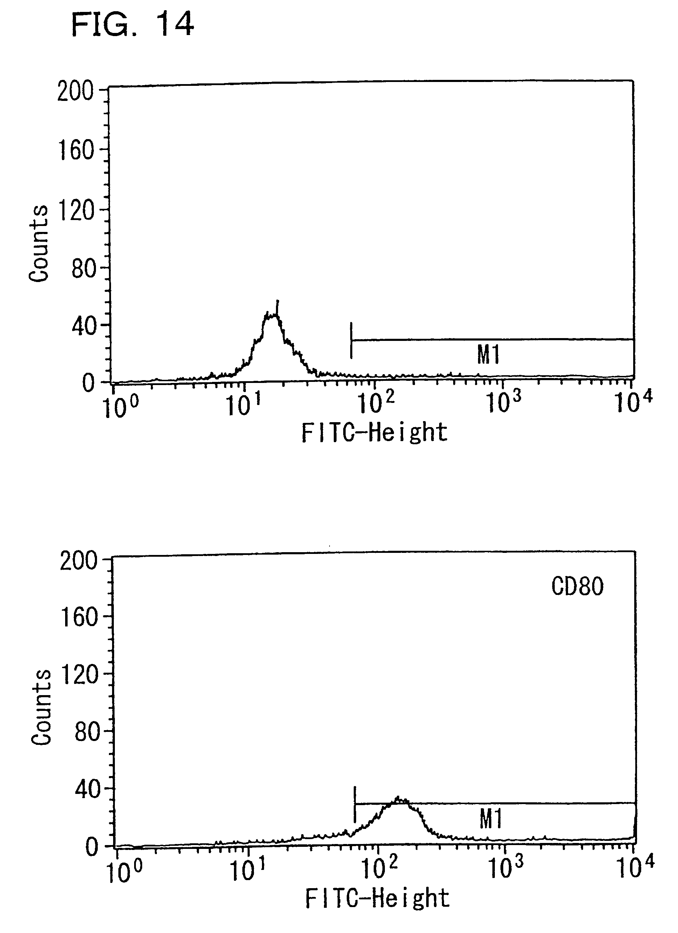

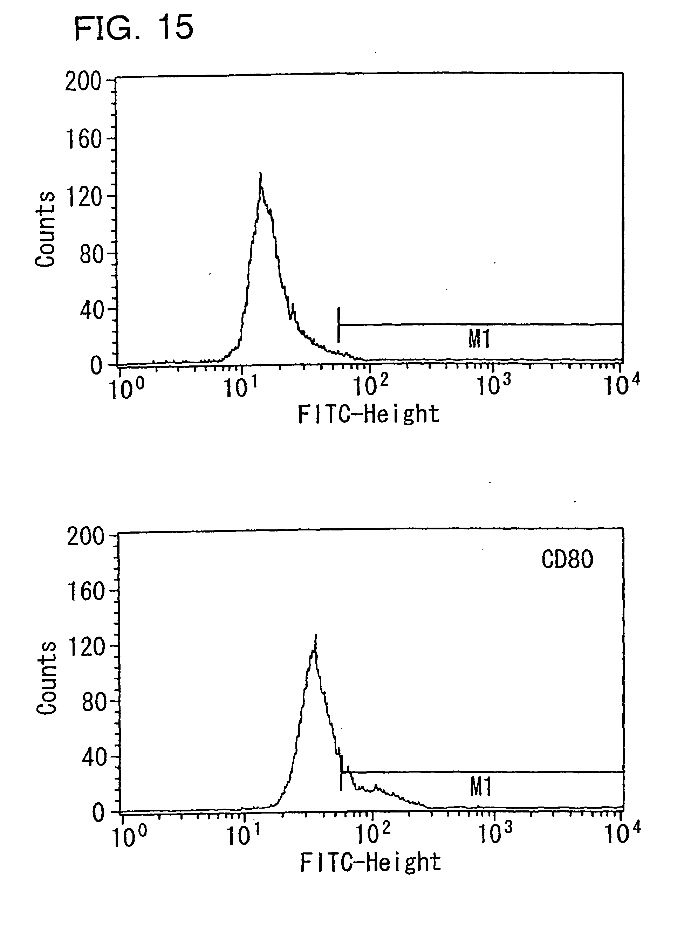

In the test samples examined, by adding ABMK0415 and ABMK6873 on the sixth day after the culture started, remarkably strong expression induction of CD80 molecules was observed. Examples of representative patterns are shown in Figures **14** and **15**.

Figure **14** shows the test results for ABMK6873. The chart shown in the upper half of Figure **14** shows the results of flow cytometry analysis of cells with no test sample added. The chart shown in the lower half of Figure **14** shows the results of flow cytometry analysis of culture cells after ABMK6873 was added. In the figure, horizontal axes indicate fluorescence strength (10 g), vertical axes indicate the number of cells, M1 indicates a region set by control antibody (IgGIFITC) measurement values.

As shown in Figure **14**, an increase in the expression of CD80 was recognized on about 92% of DCs by addition of ABMK6873 (the measured numerical values are not shown). Thus, it was indicated that activation of dendric cells was induced by addition of ABMK6873. Expression of CD80 is shown by the presence of DC in the M1 region.

Similarly, Figure **15** shows test results for ABMK0415. The chart shown in the upper half of Figure **15** shows the results of flow cytometry analysis of cells with no test sample added. The chart shown in the lower half of Figure **15** shows the results of flow cytometry analysis of culture cells after ABMK0415 was added. The horizontal axes and the vertical axes are the same as described with respect to Figure **14**.

As shown in Figure **15**, after addition of ABMK0415, an increase in expression of CD80 is recognized on about 20% of DCs (the measured numerical values are not shown). It is shown that addition of ABMK0415 induced activation of dendric cells.

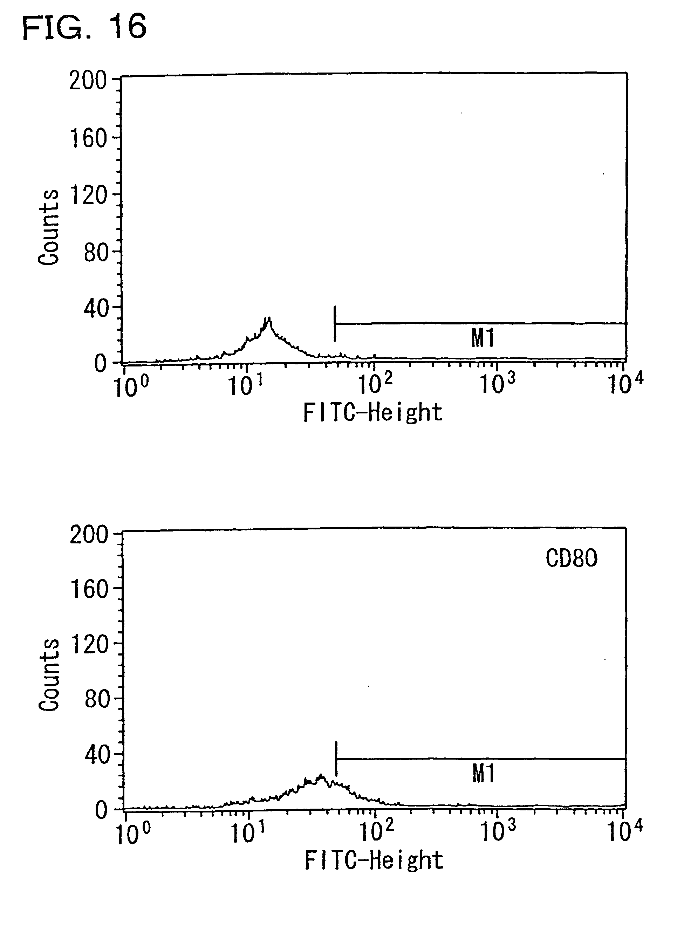

On the other hand, Figure **16** shows the results for the same PBMC, which are obtained by adding Picibanil on the sixth day after the culture of the DCs started. Picibanil is known to have DC inducing power. The chart in the upper half of Figure **16** shows the results with no Picibanil added. The chart in the lower half of Figure **16** shows that an increase in expression of CD80 is recognized on about 26% of DCs (the measured numerical values are not shown) and addition of Picibanil induced activation of dendric cells.

The results shown in Figures **14, 15**, and **16** indicate that both ABMK0415 and ABMK6873 have DC inducing power, and particularly, ABMK6873 has a DC inducing power stronger than that of Picibanil.

### INDUSTRIAL APPLICABILITY

A composition which induces apoptosis of cancer cells and health foods including the same are provided. An agaricus extract includes an ingredient which suppresses growth of leukemia cell lines and induces apoptosis. Since induction of apoptosis and induction of differentiation are effective measures in cancer treatment, a medicament and health food which are useful for cancer patients can be provided.

## Claims

1. A composition for inducing apoptosis of cells which comprises an agaricus extract.

2. A composition according to claim 1, further comprising a pharmaceutically acceptable carrier.

3. A composition according to claim 1, wherein the cells are cancer cells.

4. A composition according to claim 1, further comprising a differentiation induction agent.

5. A composition according to claim 4, wherein the differentiation induction agent is a vitamin A derivative.

6. A composition according to claim 5, wherein the vitamin A derivative is tretinoin.

7. A composition according to claim 1, wherein the agaricus extract is a dialyzate obtained by a method comprising the steps of:
extraction with hot water from a fruiting body of agaricus;
mixing the extract obtained with ethanol and centrifuging to separate precipitate and supernatant;
mixing the supernatant with ethanol and centrifuging to separate precipitate and supernatant; and
dissolving the precipitate into distilled water to perform a dialysis process.

8. A method for inducing apoptosis of cells, comprising a step of administering a composition including an agaricus extract to a subject.

9. A method according to claim 8, wherein the composition further comprises a pharmaceutically acceptable carrier.

10. A method according to claim 8, wherein the cells are cancer cells.

11. A method according to claim 8, wherein the composition further comprises a differentiation induction agent.

12. A method according to claim 11, wherein the differentiation induction agent is a,vitamin A derivative.

13. A method according to claim 12, wherein the vitamin A derivative is tretinoin.

14. A use of an agaricus extract for preparing a composition for inducing apoptosis of cancer cells.
